# EUROPEAN PATENT SPECIFICATION

(11) **EP 3 697 355 B1**
(45) Date of publication and mention of the grant of the patent: **18.12.2024**
(21) Application number: 18797141.1
(22) Date of filing: 16.10.2018
(51) Int. Cl.: A61F 9/007

(54) **SURGICAL WORKSTATION FOR SIMPLIFIED LOADING OF INTRAOCULAR LENSES**
CHIRURGISCHE ARBEITSSTATION ZUM VEREINFACHTEN LADEN VON INTRAOKULARLINSEN
POSTE DE TRAVAIL CHIRURGICAL POUR CHARGEMENT SIMPLIFIÉ DE LENTILLES INTRAOCULAIRES

(30) Priority: 18.10.2017 US 201762574112 P
(43) Date of publication of application: 26.08.2020
(73) Proprietor: Johnson & Johnson Surgical Vision, Inc., Irvine, CA 92618 (US)
(72) Inventor: KEH, Sandra, Irvine, California 92620 (US)
(74) Representative: Carpmaels & Ransford LLP
(86) International application number: PCT/IB2018/058016
(87) International publication number: WO 2019/077495

(56) References cited:
- EP-A1- 2 906 130
- WO-A1-00/24319
- WO-A1-2016/149155
- WO-A2-2008/011553
- WO-A2-2013/059719
- WO-A2-90/09141
- PT-E- 1 384 132

## Description

### BACKGROUND

### Field of Invention

The present disclosure relates generally to provide a video camera and light source to assist visualization and handling of small devices during phaco surgery.

### Description of Related Art

The invention relates generally to the field of cataract surgery, and more particularly to preparation of intraocular lenses during cataract surgery.

With age, clouding of the lens or cataracts are fairly common in humans. Cataracts may form in the central nucleus of the lens, in the softer peripheral cortical portion of the lens, or at the back of the lens near the capsular bag.

Cataracts can be treated by the replacement of the cloudy lens with an artificial lens. Phacoemulsification systems are a common device used to remove cataracts. Such systems use ultrasound energy to fragment the lens and aspirate the lens material from within the capsular bag. This may allow the capsular bag or a remnant thereof to be used to position the artificial lens (an "intraocular lens" or "IOL" as such devices are known), and maintains the separation between the anterior portion of the eye and the vitreous humour in the posterior chamber of the eye. The process of phacoemulsification of the cloudy lens and replacement of that lens with an intraocular lens is generally (and herein) referred to as "phaco surgery."

Phaco surgery is often performed by a surgeon with the aid of a surgical workstation Such Phaco systems are known from prior art documents EP2906130 or EP90/09141. Such workstation in the context of phaco surgery is usually designated a "modular ophthalmic microsurgical system" or simply a "phacoemulsification system." Such workstations typically include a surgical handpiece to facilitate cutting and emulsification (e.g., using ultrasound) of ocular tissues, fluidic connections for maintaining appropriate intraocular pressures and aspirating emulsified fragments and ocular tissues, one or more pumps (e.g., a peristaltic pump, a venture pump, or both) for facilitating fluid flow, electrical fittings for cameras and other sensors (which may or may not be part of the workstation) for viewing and sensing conditions at the surgical site, and a screen for displaying information, images, or instructions for the surgeon or other personnel. In addition to maintaining these elements together, such workstations typically are easily moved (e.g., from storage to the surgical studio) and cleaned. Connection of the various elements to a common frame facilitates keeping the elements associated with one another, so as to maintain a set of components useful in a phaco surgery procedure.

During phaco surgery, after the cataract lens has been removed, an IOL is placed in the anterior chamber of the eye. To prepare for the IOL insertion, the IOL is typically removed from the manufacturer's original package, and placed into a device known as an inserter. An inserter is a surgical instrument or tool tool used by an ocular surgeon to insert an IOL into the anterior chamber of a patient's eye through a surgical incision that has been made by the surgeon. The process of loading an IOL into an inserter is typically performed by a nurse or other professional assistant of the surgeon while the phacoemulsification procedure is being performed and before the completion of the cataract lens removal. An assistant typically hands the loaded inserter to the surgeon, and the surgeon presses on the inserter to place the IOL in the patient's eye.

The IOL is a very small piece of clear material. The dimensions of the inserter are correspondingly small (on the order of millimeters to tens of millimeters), and it can be difficult for a person loading the IOL into the inserter to align the IOL with the portion of the inserter which is to receive it and to manipulate and maneuver the IOL and the inserter sufficiently to achieve proper loading of the IOL into the inserter. Compounding these difficulties, the brightness of lights employed in cataract surgery operating rooms can be relatively dim (e.g., to enhance patient comfort and compliance and to facilitate the surgeon's ability to comfortably and accurately view the surgical field. Low light can increase eye strain and inhibit the ability of the assistant to load the IOL into the inserter.

Mis-loading or improper loading of an IOL in an inserter can damage the IOL, delay the surgery, or even result in inadvertent loss of the IOL (e.g., if it falls or drops from the IOL in an unexpected manner due to mis-loading). Such delays and/or need to obtain another IOL and properly load it can increase the duration, cost, and risk of complications in the phaco surgery. Mis-loading is therefore a significant disadvantage to be avoided if possible.

Presently, professionals tend to insert IOLs into their inserters by looking very closely and carefully at the IOL and the inserter while properly aligning the IOL with the receiving portion of the inserter, urging the IOL into the orifice surrounding the receiving portion and through that orifice into the receiving portion, and thereafter confirming loading by visually sighting the IOL within the inserter. Such professionals will sometimes employ a magnifying glass or common reading glasses while loading the IOL into the inserter. However, these devices are not designed specifically to assist this loading process and can sometimes provide optical distortions or reflections that complicate the loading. A need exists for better means for easily and reliably loading IOLs into inserters in the context of (at least) phaco surgery procedures. The subject matter described herein satisfies this need.

### SUMMARY

The disclosure relates to a surgical workstation for facilitating loading of an intraocular lens into an inserter for phaco surgery, the workstation comprising: a movable frame; an ocular surgical instrument coupled with the frame; a platform fixedly coupled with the frame and positioned gravitationally at or beneath a working area, wherein the platform is configured to support and steady the intraocular lens and the inserter while an operator loads the intraocular lens into the inserter, wherein the working area is a region of empty space in the immediate vicinity of the workstation in which an operator can perform loading of an intraocular lens into an inserter; a display coupled with the frame; an imaging system operably linked with the display and coupled with the frame at a position and in an orientation adapted to capture images of the intraocular lens and/or the inserter within the working area and to present such images upon the display in essentially real time, wherein the intraocular lens and/or the inserter on the platform surface are in clear focus when the images gathered by the imaging system are observed; and a light source coupled with the frame for illuminating the working area.

The various components can be connected to or coupled with the frame in a manner which permits their spatial movement and re-orientation relative to the frame and to one another, and electrically-powered components, such as the display, the imaging system, and the light source, can have controls operably connected or coupled therewith for adjusting operation of those components. By way of example, the brightness and sharpness of the display can be adjustable; the focus and brightness of the imaging system can be adjustable; and the intensity of the light source can be adjustable.

### BRIEF DESCRIPTION OF THE DRAWINGS

The organization and manner of the structure and function of the disclosure, together with the further objects and advantages thereof, may be understood by reference to the following description taken in connection with the accompanying drawings, and in which:
Figure 1 is an image which depicts one embodiment of the phaco surgery workstation described herein; and
Figure 2 is an image which depicts a second embodiment of the phaco surgery workstation described herein.

### DETAILED DESCRIPTION

The following description and the drawings illustrate specific embodiments sufficiently to enable those skilled in the art to practice the described system and method. Other embodiments may incorporate structural, logical, process and other changes. Examples merely typify possible variations. Individual components and functions are generally optional unless explicitly required, and the sequence of operations may vary. Portions and features of some embodiments may be included in or substituted for those of others.

The disclosure relates to a surgical workstation for phaco surgery that is adapted to facilitate loading of an IOL into an inserter. The workstation includes an imaging system (e.g., a video camera) positioned and oriented to assist a person (such as a surgeon's assistant) to visualize and, preferably, magnify the IOL, the inserter, or both in close proximity to the workstation, so as to aid the person in loading the IOL into the inserter. In one embodiment, the workstation includes a horizontal platform positioned in the field of the camera at a distance such that objects on the platform surface are in clear focus when the image gathered by the camera is observed. The platform can be used to support and steady the IOL and the inserter (and/or the operator's hands) while an operator loads the IOL into the inserter while viewing the gathered image. The workstation can further include a light source positioned to illuminate the portion of the platform within the field of view of the camera, so as to increase the ability of the operator to view the IOL and inserter without substantially affecting the level of light in the operating room that is experienced by the patient and/or the surgeon. The light source may have a shade, cover, and/or filter associated with it to reduce such affect.

The phaco surgery workstation described herein is an improvement over existing phaco workstations, which typically include a movable (e.g., wheeled or mounted upon casters) frame that carries at least an ocular surgery tool and/or instrument (e.g., a phaco surgical handpiece, such as a scalpel or ultrasonic cutter adapted with an aspirator) and fluidic components used in phaco surgery (e.g., one or more fluid pumps and sensors for detecting fluid pressures). Such workstations often include additional components, such as cameras and sensors (or electrical or wireless connectors for receiving signals from cameras or sensors) for visualizing and providing information about the surgical site to the surgeon, a display (e.g., a graphical user interface, computer monitor, common video screen, or projection screen, television screen, etc.) upon which video, photographic, or textual information can be displayed (and viewed by the surgeon or others), one or more trays, tables, or platforms upon which the surgeon or others can rest or store surgical instruments, papers, reagents, or other objects, and electrical and fluid connections to facilitate use of the workstation in a phaco surgery procedure.

The workstations describe herein include at least one additional component: an imaging system that is operably connected (or connectable) or coupled with the workstation display, directed in the immediate vicinity of the workstation (i.e., within 0 to about 182,88 cm (0 to about 72 inches) thereof, and preferably not more than about 60,96-91,44 cm (24-36 inches) therefrom, and not, for example intended to be directed at the patient or the surgical site) and able to focus in (i.e., provide clear images from) a working area that is at about normal working height for a surgeon's assistant (i.e., at least two feet, and not more than six feet, above the floor of the operating room).

The imaging system preferably includes a camera that collects and provides to a viewing screen (which can be the same screen present on current phaco surgery workstations or a separate screen mounted on the workstation) an essentially real-time image of the working area. A wide variety of cameras are known which can provide essentially real-time video images (by which is meant a series of images updated sufficiently quickly that an operator viewing the images would be substantially unable to distinguish the images of the working area from direct views of the working area), and substantially any of these cameras can be used.

The working area for which an image is collected and displayed by the imaging system should be of a size sufficient to view an IOL and an inserter as they are held and manipulated by an operator while loading the IOL into the inserter. By way of example, this working area may have dimensions (width x depth x height (above the floor)) of about 60,96 cm (24 inches) to 121,92 cm (48 inches), e.g. 60,96 cm x 60,96 cm x 30,48 cm, of 30,48 cm x 30,48 cm x 30,48 cm, 15,24 cm x 15,24 cm x 15,24 cm, or 15,24 cm x 30,48 x 15,24 cm ( 24" × 24" × 12", of 12" × 12" × 12", 6" × 6" × 6", or 6" × 12" × 6".) Characteristics of the imaging system may limit one or more dimensions of the working area, and the working area (and any components associated with it, such as a platform or light source) may be selected accordingly to facilitate viewing. The precise dimensions of the working area are not critical, other than the working area should be of a size sufficient for an operator to see the IOL as it is transferred from its manufacturer's packaging into the inserter (including any intermediate transfer from the packaging into a temporary holder or instrument). Similarly, the size of the displayed image of the working area is not critical, other than that it should be of a size sufficient to enable the operator holding the IOL and the inserter to view the working area as they are manipulated. The displayed image should also be displayed at a position and in an orientation so that the operator can view the displayed image while holding and manipulating the IOL and the inserter. The displayed image can be viewed on a display or, for example, upon a screen or other surface upon which the image is projected. The display, screen, or surface can be adjustable by the operator so as to facilitate comfortable viewing during manipulation of the IOL and the inserter. If the displayed image is shown to the operator on a display, then that display can be the same display used for other purposes (e.g. to display information to the surgeon), or it can be a separate display, such as a video display dedicated for viewing of the working area.

The display of the present invention may allow for still frame and live video that may both provide for image enhancement. Such enhancement may include magnification of at least one portion of the image and may include color scale and other optical changes which may enhance the visual clarity of the operator. By way of example, using controls provided with the system, the portion of the image most directly centered on the IOL and the inserter may be magnified by a direct focused magnification system, for example, and then presented in grey-scale, for example, to allow for greater definition of the portions of the viewed image.

The displayed image of the working area can be magnified, so that the displayed image of the objects in the work area (e.g., the IOL, the inserter, and all or portions of the operator's hands) appear larger than they actually are. Such magnification can facilitate manipulation of IOLs, which are typically very small (having maximum dimensions typically not greater than about 12-14 millimeters). In one embodiment, the magnification of the working area is adjustable by the operator and both optical and software mechanisms for magnifying video images are well known and unnecessary to describe here. Substantially any magnification control system can be employed, and the operator control of such magnification is preferably a simple device, such as a turnable knob or slidable bar.

Whether or not the presented image of the working area is presented on a display ordinarily used by the surgeon in connection with surgical operations of the workstation, the presented image should be viewable (e.g., from the position of the operator's separate display or by a copy of the image transmitted to the surgeon's display) by the surgeon, preferably without having to move from the position the surgeon occupies during surgical operations. This permits the surgeon to double-check and confirm proper loading of the IOL into the inserter prior to the surgeon's use of the inserter to insert the IOL into the patient's eye.

The working area is the region of space upon which the imaging system is focused. In one embodiment, the working area is simply a region of empty space in the vicinity of the workstation toward which the imaging system is directed and in which the operator can perform loading of an IOL into its inserter while viewing the working area on the display. Such an empty-space working area can be readily detected (e.g., by the operator waving a hand in the region of the imaging system to visually detect the location of the working area before performing IOL manipulations). Alternatively, the working area can be described, such as by symbols or text visible on a surface of the workstation.

In a preferred embodiment, the working area is situated on or just above a platform that is preferably attached to the work station. The platform can, for example, be flat and horizontally oriented, to facilitate manipulation of the IOL and the inserter upon the platform surface. The platform can alternatively have a concave shape (such that any dropped object will tend to fall or roll toward a central part of the platform) or any other desired shape (such as having a fitting with a shape selected to rigidly hold the inserter during IOL loading). The platform can be rigidly attached to the workstation or it can movably or adjustably attached. By way of example, the platform can be secured by a threaded rod which extends through a portion of the platform and impacts a part of the frame of the workstation so that the platform can be moved (e.g., vertically to facilitate a height comfortable to the operator or laterally to position the platform at a location convenient for the operator or the surgeon) by loosening and tightening the threaded rod against the frame. If the platform is adjustable, the imaging system can be fixedly attached to or coupled with the platform, so that the imaging system remains focused on the working area on the platform without the need to move, adjust, or refocus the imaging system. Alternatively, the platform and the imaging system can be separately movable by the operator, so as to facilitate use of a preferred section of the platform as the working area (e.g., if a different section of the platform is being employed for another purpose).

The workstation can further include a light source for illuminating the working area. Such illumination can assist the operator to view the working area (e.g., directly or by way of a display). The identity and type of light source is not critical and can be, for example, an incandescent lamp, a halogen lamp, a light-emitting diode (LED) or LED array, or a mirror for directing ambient light from the operating room onto the working area. Likewise, the light source can have a single operating intensity or the intensity can be adjustable (e.g., with a dimmer switch or a computer-operated control). The light source should be located (preferably attached to the workstation) at position and in an orientation that it will emit light onto (i.e., in the direction of) the working area from a direction in which the emitted light will not be obscured by any of the operator's hands, the IOL and its packaging, and the inserter. By way of example, if the workstation includes a platform upon which the working area is situated, the platform being attached to the workstation frame, and the operator normally manipulating the IOL and insert on the opposite side of the platform from its attachment point, then the light source should be mounted on the frame on the side of the platform opposite the operator. The light source should also be positioned and oriented such that light emitted into, and reflected by objects from, the working area can be expected to be captured by the imaging system.

Light emitted by the light source can have any spectral content, so long as light collected by the imaging system is displayed to the operator in images that are visually perceptible by the operator. Thus, for example, if the IOL includes a material that emits fluorescent light at a wavelength outside the human visible spectrum, that non-visible light can be captured by the imaging system and translated on the display to a wavelength that is visible to the operator, enabling the operator to better view the IOL. Similarly, light fluorescently emitted or reflected by portions of the inserter (e.g., the boundaries of an orifice leading to the receiving portion) can be visualized, further facilitating loading.

The light source can be fixed to or coupled with the workstation frame, to the imaging system, to the platform, or to none of these. The light source can be positionally adjustable by the operator, such as by means of an articulable "gooseneck" type connector common in adjustable lamps.

In another embodiment, the image system may capture a barcode that contains data, such as, pictures, text, numbers, signatures, and/or graphics, that is presented into the field of view of the image system and/or light source. The image system and/or light source may include one or more barcode readers/scanners to enable the reading of one or more barcodes. The barcode may be any barcode known in the art, including, but not limited to UPC, EAN, QR, Code 39, CODABAR, DATAMATRIX CODE, PDF417, and similar codes thereof. The data contained in the code may be thereafter stored in the memory of the phacoemulsification system, transferred to an external storage, database or cloud location, and/or presented on the display.

### Examples

The subject matter of this disclosure is now described with reference to the following Examples. These Examples are provided for the purpose of illustration only, and the subject matter is not limited to these Examples, but rather encompasses all variations which are evident as a result of the teaching provided herein.

### Example 1

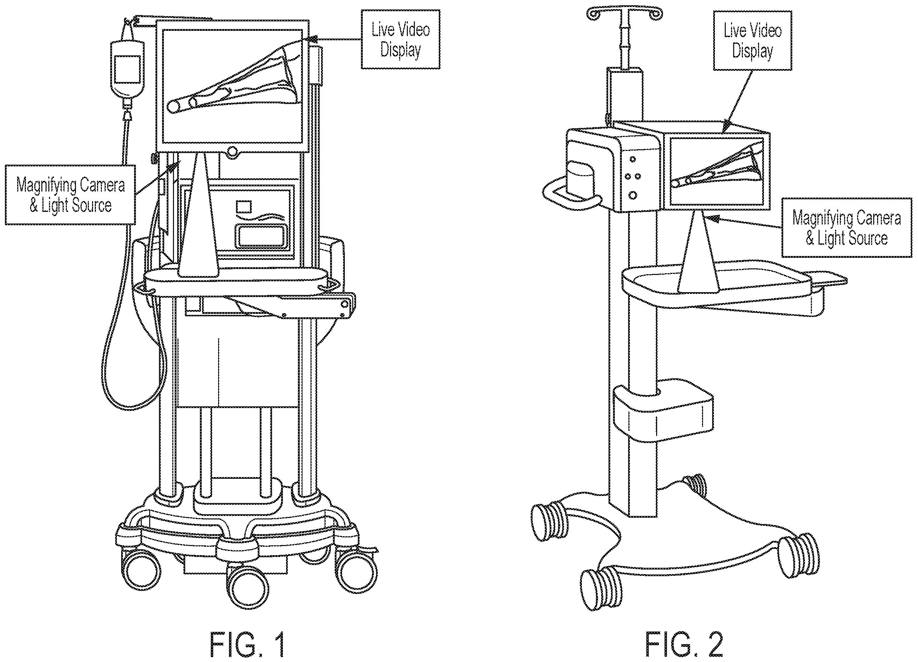

Figure 1 is an image which depicts one embodiment of the phaco surgery workstation described herein. In this embodiment, a wheeled frame carries a video monitor on which a live video image can be observed, the image being captured in real time by a video camera mounted behind the video screen in this view and directed downward toward the surface of a horizontal platform also carried by the wheeled frame. Also mounted behind and near the lower extremity of the video monitor is a light source which can be seen to project visible light onto the horizontal platform.

In this embodiment, an operator is able to load an IOL into an inserter in the working area, which is the illuminated area atop the horizontal platform. The operator stands in front of (in the view shown in Figure 1) the workstation, facing the workstation. The operator manipulates the IOL and the inserter atop the platform in the illuminated area, opening the IOL manufacturer's packaging and loading the IOL thence into the inserter. The imaging system displays the manipulations as they occur on the video screen. By watching the video screen during the manipulations, loading of the IOL into the inserter by the operator is facilitated. As illustrated in Figure 1, the image of the working area is magnified on the video screen.

### Example 2

Figure 2 is an image which depicts a second embodiment of the phaco surgery workstation described herein. In this embodiment, a frame is mounted upon casters to facilitate its movement. The frame carries a unit that includes a video monitor on which a live video image can be observed, the image being captured in real time by a video camera mounted on the bottom of the unit in this view and directed downward toward the surface of a horizontal platform also carried by the wheeled frame. The unit also includes a light source which can be seen to project visible light onto the horizontal platform. Each of the unit and the horizontal platform is separately vertically adjustable, to facilitate the operator's comfort in viewing the video monitor and in manipulating objects upon the horizontal platform.

In this embodiment, an operator is able to load an IOL into an inserter in the working area, which is the illuminated area atop the horizontal platform. The operator stands in front of (in the view shown in Figure 2) the workstation, facing the workstation. The operator manipulates the IOL and the inserter atop the platform in the illuminated area, opening the IOL' manufacturer's packaging and loading the IOL thence into the inserter. The imaging system displays the manipulations as they occur on the video screen. By watching the video screen during the manipulations, loading of the IOL into the inserter by the operator is facilitated. As illustrated in Figure 2, the image of the working area is magnified on the video screen.

While this subject matter has been disclosed with reference to specific embodiments, it is apparent that other embodiments and variations can be devised by others skilled in the art without departing from the scope of the subject matter described herein. The appended claims include all such embodiment .

## Claims

1. A surgical workstation for facilitating loading of an intraocular lens into an inserter for phaco surgery, the workstation comprising:
a movable frame;
an ocular surgical instrument coupled with the frame;
a platform fixedly coupled with the frame and positioned gravitationally at or beneath a working area, wherein the platform is configured to support and steady the intraocular lens and the inserter while an operator loads the intraocular lens into the inserter, wherein the working area is a region of empty space in the immediate vicinity of the workstation in which an operator can perform loading of an intraocular lens into an inserter;
a display coupled with the frame;
**characterised by**
an imaging system operably linked with the display and coupled with the frame at a position and in an orientation adapted to capture images of the intraocular lens and/or the inserter within the working area and to present such images upon the display in essentially real time, wherein the intraocular lens and/or the inserter on the platform surface are in clear focus when the images gathered by the imaging system are observed; and
a light source coupled with the frame for illuminating the working area.

2. The surgical workstation of claim 1, comprising an operable control for at least one of the intensity, the position, and the direction of illumination of the light source.

3. The surgical workstation of claim 1, wherein the field of view of the imaging system is sufficiently broad to capture images of an intraocular lens and an inserter as they are being manipulated by an operator of the workstation.

4. The surgical workstation of claim 1, wherein the platform is oriented substantially horizontally, relative to gravity.

5. The surgical workstation of claim 1, wherein the imaging system comprises a video camera.

6. The surgical workstation of claim 5, wherein the workstation comprises an operable focus control whereby an operator of the workstation can adjust the focus of the video camera.

7. The surgical workstation of claim 5, wherein the workstation comprises an operable control whereby an operator of the workstation can adjust at least one of the position and the orientation of the imaging system.

8. The surgical workstation of claim 1, wherein the working area is situated within 183 cm (72 inches) of the frame and at least 61 cm (two feet) above the floor upon which the frame rests.

9. The surgical workstation of claim 1, wherein the working area has dimensions of 15 cm (6 inches) width, by 15 cm (6 inches) depth, by 15 cm (6 inches) height.

10. The surgical workstation of claim 1, wherein the imaging system, the display, and their operable connection are selected so as to present on the display a magnified image of the intraocular lens and/or the inserter in the working area.

11. The surgical workstation of claim 10, wherein the workstation comprises an operable control whereby an operator of the workstation can adjust the degree of magnification of the magnified image presented on the display.

12. The surgical workstation of claim 1, wherein
the imaging system is coupled with the frame at a position and in an orientation adapted to capture a barcode associated with the intraocular lens and/or the inserter if said barcode is presented in a field-of-view of the imaging system.

13. The surgical workstation of claim 12, wherein the barcode is selected from the group consisting of UPC, EAN, QR, Code 39, CODABAR, DATAMATRIX CODE, and PDF417.

14. The surgical workstation of claim 12, wherein the imaging system comprises a barcode reader.

## Patentansprüche

1. Chirurgische Arbeitsstation zum Erleichtern eines Ladens einer Intraokularlinse in einen Inserter für eine Phako-Chirurgie, die Arbeitsstation umfassend:
einen bewegbaren Rahmen;
ein augenchirurgisches Instrument, das mit dem Rahmen gekoppelt ist;
eine Plattform, die mit dem Rahmen fest gekoppelt und gravitativ an oder unter einem Arbeitsbereich positioniert ist, wobei die Plattform konfiguriert ist, um die Intraokularlinse und den Inserter zu stützen und zu stabilisieren, während ein Bediener die Intraokularlinse in den Inserter lädt, wobei der Arbeitsbereich eine Region eines leerer Raums in unmittelbarer Nähe der Arbeitsstation ist, in dem ein Bediener das Laden einer Intraokularlinse in einen Inserter durchführen kann;
ein Display, das mit dem Rahmen gekoppelt ist;
**gekennzeichnet durch**
ein Bildgebungssystem, das mit dem Display wirkverbunden und mit dem Rahmen an einer Position und in einer Ausrichtung gekoppelt ist, die angepasst ist, um Bilder der Intraokularlinse und/oder des Inserters innerhalb des Arbeitsbereichs aufzunehmen und um derartige Bilder im Wesentlichen in Echtzeit auf dem Display darzustellen, wobei die Intraokularlinse und/oder der Inserter auf der Plattformoberfläche klar fokussiert sind, wenn die Bilder betrachtet werden, die durch das Bildgebungssystem erfasst wurden; und
eine Lichtquelle, die mit dem Rahmen zum Beleuchten des Arbeitsbereichs gekoppelt ist.

2. Chirurgische Arbeitsstation nach Anspruch 1, umfassend eine bedienbare Steuerung für mindestens eines von der Intensität, der Position und der Beleuchtungsrichtung der Lichtquelle.

3. Chirurgische Arbeitsstation nach Anspruch 1, wobei das Sichtfeld des Bildgebungssystems ausreichend breit ist, um Bilder einer Intraokularlinse und eines Inserters aufzunehmen, während diese durch einen Bediener der Arbeitsstation manipuliert werden.

4. Chirurgische Arbeitsstation nach Anspruch 1, wobei die Plattform im Wesentlichen horizontal im Verhältnis zu der Schwerkraft ausgerichtet ist.

5. Chirurgische Arbeitsstation nach Anspruch 1, wobei das Bildgebungssystem eine Videokamera umfasst.

6. Chirurgische Arbeitsstation nach Anspruch 5, wobei die Arbeitsstation eine bedienbare Fokussteuerung umfasst, wodurch ein Bediener der Arbeitsstation den Fokus der Videokamera anpassen kann.

7. Chirurgische Arbeitsstation nach Anspruch 5, wobei die Arbeitsstation eine bedienbare Steuerung umfasst, wodurch ein Bediener der Arbeitsstation mindestens eines von der Position und der Ausrichtung des Bildgebungssystems anpassen kann.

8. Chirurgische Arbeitsstation nach Anspruch 1, wobei sich der Arbeitsbereich innerhalb von 183 cm (72 Zoll) des Rahmens und mindestens 61 cm (zwei Fuß) über dem Boden befindet, auf dem der Rahmen ruht.

9. Chirurgische Arbeitsstation nach Anspruch 1, wobei der Arbeitsbereich Abmessungen von 15 cm (6 Zoll) Breite mal 15 cm (6 Zoll) Tiefe mal 15 cm (6 Zoll) Höhe aufweist.

10. Chirurgische Arbeitsstation nach Anspruch 1, wobei das Bildgebungssystem, das Display und ihre betriebsfähige Verbindung ausgewählt sind, sodass auf dem Display ein vergrößertes Bild der Intraokularlinse und/oder des Inserters in dem Arbeitsbereich dargestellt wird.

11. Chirurgische Arbeitsstation nach Anspruch 10, wobei die Arbeitsstation eine bedienbare Steuerung umfasst, wodurch ein Bediener der Arbeitsstation den Vergrößerungsgrad des vergrößerten Bilds anpassen kann, das auf dem Display dargestellt wird.

12. Chirurgische Arbeitsstation nach Anspruch 1, wobei das Bildgebungssystem an einer Position und in einer Ausrichtung mit dem Rahmen gekoppelt ist, die angepasst sind, um einen Strichcode aufzunehmen, der mit der Intraokularlinse und/oder dem Inserter verknüpft ist, falls der Strichcode in einem Sichtfeld des Bildgebungssystems dargestellt wird.

13. Chirurgische Arbeitsstation nach Anspruch 12, wobei der Barcode aus der Gruppe ausgewählt ist, bestehend aus UPC, EAN, QR, Code 39, CODABAR, DATAMATRIX CODE und PDF417.

14. Chirurgische Arbeitsstation nach Anspruch 12, wobei das Bildgebungssystem einen Barcodeleser umfasst.

## Revendications

1. Poste de travail chirurgical permettant de faciliter le chargement d'une lentille intraoculaire dans dispositif d'insertion pour une phaco-chirurgie, le poste de travail comprenant :
un châssis mobile ;
un instrument chirurgical oculaire accouplé au châssis ;
une plate-forme accouplée fixement au châssis et positionnée par gravité au niveau ou en dessous d'une zone de travail, dans lequel la plate-forme est conçue pour supporter et stabiliser la lentille intraoculaire et le dispositif d'insertion alors qu'un opérateur charge la lentille intraoculaire dans le dispositif d'insertion, dans lequel la zone de travail est une région d'espace vide au voisinage immédiat du poste de travail dans laquelle un opérateur peut mettre en oeuvre un chargement d'une lentille intraoculaire dans un dispositif d'insertion ;
un affichage accouplé au châssis ;
**caractérisé par**
un système d'imagerie lié de manière fonctionnelle à l'affichage et accouplé au châssis au niveau d'une position, et dans une orientation, conçues pour capturer des images de la lentille intraoculaire et/ou du dispositif d'insertion au sein de la zone de travail et pour présenter de telles images sur l'affichage sensiblement en temps réel, dans lequel la lentille intraoculaire et/ou le dispositif d'insertion sur la surface de plate-forme sont clairement mis au point lorsque les images rassemblées par le système d'imagerie sont observées ; et
une source de lumière accouplée au châssis permettant d'illuminer la zone de travail.

2. Poste de travail chirurgical selon la revendication 1, comprenant une commande fonctionnelle pour au moins l'une parmi l'intensité, la position et la direction d'illumination de la source de lumière.

3. Poste de travail chirurgical selon la revendication 1, dans lequel le champ de vision du système d'imagerie est suffisamment large pour capturer des images d'une lentille intraoculaire et d'un dispositif d'insertion à mesure qu'ils sont manipulés par un opérateur du poste de travail.

4. Poste de travail chirurgical selon la revendication 1, dans lequel la plate-forme est orientée de façon sensiblement horizontale, par rapport à la gravité.

5. Poste de travail chirurgical selon la revendication 1, dans lequel le système d'imagerie comprend une caméra vidéo.

6. Poste de travail chirurgical selon la revendication 5, dans lequel le poste de travail comprend une commande de mise au point fonctionnelle par laquelle un opérateur du poste de travail peut ajuster la mise au point de la caméra vidéo.

7. Poste de travail chirurgical selon la revendication 5, dans lequel le poste de travail comprend une commande fonctionnelle par laquelle un opérateur du poste de travail peut ajuster au moins l'une parmi la position et l'orientation du système d'imagerie.

8. Poste de travail chirurgical selon la revendication 1, dans lequel la zone de travail est située à moins de 183 cm (72 pouces) du châssis et au moins 61 cm (deux pieds) au-dessus du fond sur lequel repose le châssis.

9. Poste de travail chirurgical selon la revendication 1, dans lequel la zone de travail a des dimensions de 15 cm (6 pouces) de largeur, sur 15 cm (6 pouces) de profondeur, sur 15 cm (6 pouces) de hauteur.

10. Poste de travail chirurgical selon la revendication 1, dans lequel le système d'imagerie, l'affichage et leur connexion fonctionnelle sont sélectionnés de façon à présenter sur l'affichage une image agrandie de la lentille intraoculaire et/ou du dispositif d'insertion dans la zone de travail.

11. Poste de travail chirurgical selon la revendication 10, dans lequel le poste de travail comprend une commande fonctionnelle par laquelle un opérateur du poste de travail peut ajuster le degré d'agrandissement de l'image agrandie présentée sur l'affichage.

12. Poste de travail chirurgical selon la revendication 1, dans lequel le système d'imagerie est accouplé au châssis au niveau d'une position, et dans une orientation, conçues pour capturer un code-barres associé à la lentille intraoculaire et/ou au dispositif d'insertion si ledit code-barres est présenté dans un champ de vision du système d'imagerie.

13. Poste de travail chirurgical selon la revendication 12, dans lequel le code-barres est choisi dans le groupe constitué par UPC, EAN, QR, Code 39, CODABAR, DATAMATRIX CODE, et PDF417.

14. Poste de travail chirurgical selon la revendication 12, dans lequel le système d'imagerie comprend un lecteur de code-barres.
